# EUROPEAN PATENT APPLICATION

(11) **EP 2 764 875 A1**
(43) Date of publication of application: **13.08.2014**
(21) Application number: 14153475.0
(22) Date of filing: 31.01.2014
(51) Int. Cl.: A61K 47/10, A61K 9/08, A61K 31/19

(54) **Process for preparing a stable pharmaceutical composition comprising dexketoprofen trometamol**

(30) Priority: 06.02.2013 EP 13154281
(71) Applicant: Galenicum Health S.L., 08005 Barcelona (ES)
(72) Inventor: Pérez Cadahía, Beatriz, 08005 Barcelona (ES)
(74) Representative: Marjanet Artigas, Georgina

(57) **Abstract**

The present invention relates to a process for preparing a pharmaceutical composition in the form of stick-pack comprising dexketoprofen trometamol, and the pharmaceutical composition obtained according to said process. The process provides a stable liquid pharmaceutical composition, with a low content of impurities and with excellent properties to be filled into stick-packs.

## Description

The present invention relates to a process for preparing a pharmaceutical composition in the form of stick-pack comprising dexketoprofen trometamol, and the pharmaceutical composition obtained according to said process. The process provides a stable liquid pharmaceutical composition, with a low content of impurities and with excellent properties to be filled into stick-packs.

### STATE OF THE ART

Dexketoprofen trometamol or (+)-(S)-2-(3-benzoylphenyl)propionic acid tromethamine salt, belongs to a class of medicines called non-steroidal anti-inflammatory drugs (NSAIDs). The empirical formula of dexketoprofen trometamol is C16H14O3.C4H11NO3 and the compound has a molecular weight of 375.42. The structural formula of dexketoprofen trometamol is (I):

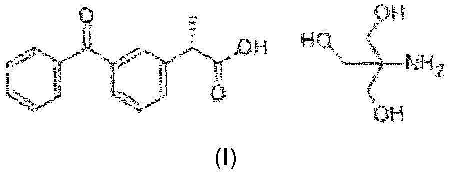

Dexketoprofen works by blocking the action of an enzyme in the body called cyclooxygenase. Cyclooxygenase is involved in the production of chemicals in the body called prostaglandins. Prostaglandins are produced in response to injury or certain diseases and would otherwise go on to cause swelling, inflammation and pain. By blocking cyclooxygenase, dexketoprofen prevents the production of prostaglandins and therefore reduces inflammation and pain. Along with peripheral analgesic action it possesses central analgesic action.

Dexketoprofen was first disclosed in the WO8904658. The WO9411332 relates to a specific salt of dexketoprofen, concretely dexketoprofen trometamol salt. However, both WO8904658 and WO9411332 are silent on how to specifically formulate either dexketoprofen or dexketoprofen trometamol.

Convenience of administration and patient compliance are gaining significant importance in the design of dosage forms. Recently, more stress is laid down on the development of an organoleptically elegant and patient-friendly drug delivery system for pediatric and geriatric patients. More than 50% of the pharmaceutical products are orally administered for several reasons, and undesirable taste is one of the important formulation problems encountered with such oral products. Taste of a pharmaceutical product is an important parameter for governing compliance. Thus, taste masking of oral pharmaceuticals has become an important tool to improve patient compliance and the quality of treatment, especially in pediatrics. Therefore, formulation of taste-masked products is a challenge to the pharmacists.

Solutions and suspensions of drugs are used widely in the pharmaceutical industry for production of dosage forms for different routes of administration; for example, oral, parenteral and inhalation. The manufacture and control of oral solutions and oral suspensions presents some unusual problems not common to other dosage forms. Although bioequivalence concerns are minimal (except for product in which dissolution is a rate-limiting or an absorption-determining step), other issues have frequently led to recalls of liquid products. These include microbiological, potency, dose uniformity and stability problems. Deterioration of an oral liquid may be due to chemical, physical or microbiological instability which can lead to a subtherapeutic dose of drug, exposure to toxic degradation products or ingestion of unacceptable numbers of microorganisms. In addition, because the population using these oral dosage forms includes newborns, pediatrics, and geriatrics, who may not be able to take oral solid dosage forms and who may have compromised drug metabolic or other clearance function, defective dosage forms can pose a greater risk if the absorption profiles are significantly altered from the profiles used in the development of drug safety profiles.

Thus, there is still a need in the art, in order to improve patient compliance, to develop liquid pharmaceutical compositions being stable, having dose uniformity and having microbiological problems minimized.

### SUMMARY OF THE INVENTION

The present invention provides an oral stable pharmaceutical composition in the form of stick-pack comprising dexketoprofen trometamol.

The composition as herein disclosed of dexketoprofen trometamol is stable in spite of the use of a thin aluminium layer in the stick package. In addition, the composition according to the present invention offers the advantage of being easily manipulable and easy to be opened due to the thickness of the aluminium used in the packaging. The present invention also provides with a low content of impurities from the decomposition of dexketoprofen trometamol. It is also provided a manufacturing processes for the manufacture of solutions comprising dexketoprofen trometamol wherein provides a solution with a low content of impurities and with excellent properties to be filled into stick-packs.

In a first aspect, it is provided a pharmaceutical composition in the form of stick-pack comprising dexketoprofen trometamol, wherein the pharmaceutical composition comprises at least the following two portions:
i) a filling solution, wherein the filling solution comprising dexketoprofen trometamol and at least a pharmaceutically acceptable excipient or excipients, wherein the dexketoprofen trometamol is dissolved in the filling solution and the pH of the filling solution is lower than 9;
ii) a stick package, wherein the stick package comprises at least a sealed laminated foil of aluminium of less than 20.0 microns thick.

In a second aspect, it is provided a process for the manufacture of a pharmaceutical composition, wherein the process comprises at least the following steps:
a) having a first mixture of at least a pharmaceutically acceptable excipient or excipients, and water at a temperature higher than 35 °C, preferably between 36 and 75 °C, wherein the pharmaceutically acceptable excipient or excipients being preferably selected from viscosity agents, preservatives, sweetness enhancers and mixtures thereof;
b) optionally, diluting the first mixture with water;
c) preferably keeping or, if necessary, cooling the temperature of the first mixture in step (a) or of the diluted mixture in step (b) below 45 °C;
d) adding dexketoprofen trometamol, or a mixture of dexketoprofen trometamol and a further pharmaceutically acceptable excipient or excipients, to the mixture of step (a), (b) or (c);
e) stirring the mixture obtained in the preceding step until a filling solution is obtained;
f) optionally, measuring the pH of the filling solution, and adjusting to a pH below 9 if required;
g) packaging the filling solution in a stick package, wherein the stick package comprises at least a sealed laminated foil of aluminium of less than 20.0 microns thick, preferably the temperature of the filling solution is at room temperature.

In a third aspect, it is provided a filling solution comprising dexketoprofen trometamol, wherein the dexketoprofen trometamol is dissolved and the pH of the filling solution is lower than 9; wherein the filling solution is obtained by the process of the second aspect.

In a fourth aspect, it is provided a pharmaceutical batch of at least 15,000 units of the pharmaceutical composition of the first aspect.

In a fifth aspect, it is provided a cardboard box with a patient information leaflet in pack size of at least 4 stick-pack as defined in the first aspect.

### DETAILED DESCRIPTION OF THE INVENTION

In a further embodiment of the pharmaceutical composition as herein disclosed, the sealed laminated foil of aluminium of the stick package is from 5.0 to 14.0 microns thick, preferably from 7.0 to 12.0 microns thick, more preferably less than 11.0 microns thick, and even more preferably less than 10.0 microns thick.

In a further embodiment of the pharmaceutical composition as herein disclosed, the sealed laminated sheet of aluminium of the stick package is about 9 microns thick. The pharmaceutical compositions as herein disclosed showed a good stability even being packaged in a stick-pack with a thin aluminium layer.

In a further embodiment of the pharmaceutical composition as herein disclosed, the pH of the filling solution is about 3 to 8. Preferably, the pH of the filling solution is about 4.0 to 7.5. More preferably, the pH of the filling solution is about 5.5 to 6.5.

The determination of pH is measured by the method described in European Pharmacopoeia 7.0th edition 2.2.3. (Potentiometric determination of pH). The pH is a number which represents conventionally the hydrogen ion concentration of an aqueous solution. The potentiometric determination of pH is made by measuring the potential difference between 2 appropriate electrodes immersed in the solution to be examined.

In a further embodiment of the pharmaceutical composition as herein disclosed, the stick package comprises at least an internal layer and preferably the internal layer is of polyethylene. In a further embodiment, the stick package comprises at least an external layer and preferably the external layer is of polyethylene. In a further embodiment, the stick package comprises at least an external layer and at least an internal layer and preferably the stick package comprises at least a polyethylene external layer and at least a polyethylene internal layer. In a further embodiment, the stick package comprises at least an outer layer and preferably the outer layer is printed paper or polyethylene terephthalate. In a further embodiment, the stick package comprises at least an outer layer and preferably the outer layer is polyethylene terephthalate. In a further embodiment, the stick package consists essentially of printed paper foil/polyethylene foil/aluminium foil/polyethylene foil. In a further embodiment, the stick package consists essentially of polyethylene terephthalate foil/polyethylene foil/aluminium foil/polyethylene foil. Four layered foils make the formulation stable and convenient to package it.

Preferably, the product is packaged in flexible laminate stick-packs composed of printed paper/polyethylene/aluminium foil/polyethylene. More preferably, the product is packaged in flexible laminate stick-packs composed of polyethylene terephthalate/polyethylene/aluminium foil/polyethylene. The stick-pack can be supplied in cardboard boxes with patient information leaflet in pack size of at least 4 stick-packs, preferably in cardboard boxes of 20 stick-packs.

As used herein, "sachet" or "stick-pack" refers to a small, sealed packet containing a quantity of material, which is a single-use or unit dose quantity.

A packaging of the stick-pack type,_preferably with improved opening, comprises normally: a flexible film, having at least one layer, which forms a hermetically sealed tubular body with mutually opposite longitudinal film flaps, a first band provided longitudinally to said body for inside/outside sealing of said mutually opposite longitudinal flaps of the film; second sealing bands provided transversely to said body for inside/outside sealing; a sealed extension region protruding from at least one of said second sealing bands on a respective portion of at least one edge of said tubular body; and preferably a transverse pre-weakening incisions that are provided in longitudinal alignment with said sealed extension region, along at least one of said mutually opposite longitudinal flaps. The WO9501921 discloses stick-packs for the present invention, which content is herein included by reference.

In a further embodiment of the pharmaceutical composition as herein disclosed, the concentration of dexketoprofen trometamol is about 0.5 to 10 mg/ml, preferably about 1 to 5 mg/ml, more preferably about 2.5 mg/ml.

In a further embodiment of the pharmaceutical composition as herein disclosed, the volume of the filling solution is from about 5 to 20 ml, preferably from 7 to 15 ml, more preferably from 9 to 12 ml and, even more preferably about 10 ml.

In a further embodiment of the pharmaceutical composition as herein disclosed, it comprises at least a viscosity agent, a preservative, a sweetness enhancer and, if required a pH modifier agent.

In a further embodiment of the pharmaceutical composition as herein disclosed, the viscosity agent is selected from povidone, polyethylene glycol, and mixtures thereof.

In a further embodiment of the pharmaceutical composition as herein disclosed, the total amount of viscosity agent or viscosity agents, preferably povidone, polyethylene glycol and mixtures thereof, is below 60% w/w, preferably ranges from 10 to 50% w/w, more preferably from 15 to 45% w/w in respect of the total amount of the pharmaceutical composition. The pharmaceutical compositions as herein disclosed showed a good stability even being packaged in a stick-pack with a thin aluminium layer and having high amounts of viscosity agent or viscosity agents.

In a further embodiment of the pharmaceutical composition as herein disclosed, the amount of povidone is below 40% w/w, preferably ranges from 5 to 35% w/w, more preferably from 12 to 25% w/w in respect of the total amount of the pharmaceutical composition. In a further embodiment of the pharmaceutical composition as herein disclosed, the amount of polyethylene glycol is below 40% w/w, preferably ranges from 5 to 35% w/w, more preferably from 15 to 30% w/w in respect of the total amount of the pharmaceutical composition.

The term "viscosity agent" as used herein refers to a substance that when added to a liquid or semi-solid material increases the viscosity thereof.

In a further embodiment of the pharmaceutical composition as herein disclosed, the preservative is selected from salicylic acid, benzoic acid, methyl paraben, ethyl paraben, cresol, cetrimide, citric acid, EDTA and mixtures thereof. Preferably, the preservative agent is methyl paraben.

In a further embodiment of the pharmaceutical composition as herein disclosed, the amount of preservative agent, preferably methyl paraben, is_below 4% w/w, preferably ranges from 0.2 to 2% w/w, more preferably from 0.4 to 1% w/w in respect of the total amount of the pharmaceutical composition.

The term "preservative" as used herein refers to compounds which are used in pharmaceutical chemistry to prevent the growth of bacteria, mold and other microbes. They are used in the manufacture of pharmaceutical drugs and cosmetics, for their individual antibacterial (destroying and inhibiting the growth of bacteria), antifungal (destroying and inhibiting the growth of fungus) properties, anti-microbial, anti-mycoplasmal, anti-viral and/or anti-prion. A preservative must be pharmaceutically acceptable at the concentrations used, and should not interfere with the action of the active compound in the formulation. An "effective amount" of a preservative is that amount necessary to prevent the growth of microorganisms in the formulation. Suitable preservatives can include, but are not limited to, at least one of a benzalkonium chloride, a benzethonium chloride, a chlorohexidine, a phenol, a m-cresol, a benzyl alcohol, an alkyl paraben (methylparaben, ethylparaben, propylparaben, butylparaben, and the like), sodium dehydroacetate, an o-cresol, a p-cresol, a chlorocresol, a phenylmercuric nitrate, a thimerosal, a benzoic acid and any mixture thereof of one or more preservatives. See, e.g., Wallhauser, K., Develop. Biol. Standard. 24, pp. 9-28 (Basel, S. Krager, 1974).

In a further embodiment of the pharmaceutical composition as herein disclosed, the sweetness enhancer is selected from sucralose, acesulfame potassium or other salts, aspartame, alitame, sodium saccharin or other salts, neohesperidin dihydrochalcone, cyclamate, neotame, N-[3-(3-hydroxy-4-methoxyphenyl)propyl]-L-alpha.-aspartyl]-L-phenylalanine 1-methyl ester, N-[3-(3-hydroxy-4-methoxyphenyl)-3-methylbutyl]-L-alpha-aspartyl]-phenylalanine 1-methyl ester, N-[3-(3-methoxy-4-hydroxyphenyl)propyl]-L-alpha-aspartyl]-L-phenylalanine 1-methyl ester, ammonium glycyrrhizate and mixtures thereof. Preferably, the sweetness enhancer is neohesperidin dihydrochalcone, sodium saccharin, ammonium glycyrrhizate or mixtures thereof.

In a further embodiment of the pharmaceutical composition as herein disclosed, the total amount of sweetness enhancer or sweetness enhancers, preferably neohesperidin dihydrochalcone, sodium saccharin, ammonium glycyrrhizate and mixtures thereof, is_below 2% w/w, preferably below 1% w/w, more preferably below 0.3% w/w in respect of the total amount of the pharmaceutical composition.

The term "sweetness enhancer" as used herein includes compounds capable of enhancing or intensifying the perception of sweet taste of sweetener compositions or sweetened compositions. The term sweetness enhancer is synonymous with the terms "sweet taste potentiator," "sweetness potentiator," and "sweetness intensifier". Generally, the sweetness enhancers provided herein enhance the sweet taste of sweeteners without providing any noticeable sweet taste by themselves at acceptable use levels; however, the sweetness enhancers may themselves provide sweet taste at higher concentrations. Suitable sweetness enhancers can include, but are not limited to, at least one of sucralose, acesulfame potassium or other salts, aspartame, alitame, sodium saccharin or other salts, neohesperidin dihydrochalcone, cyclamate, neotame, N-[3-(3-hydroxy-4-methoxyphenyl)propyl]-L-alpha-aspartyl]-L-phenylalanine 1-methyl ester, N-[3-(3-hydroxy-4-methoxyphenyl)-3-methylbutyl]-L-alpha-aspartyl]-phenylalanine 1-methyl ester, N-[3-(3-methoxy-4-hydroxyphenyl)propyl]L-alpha-aspartyl]-L-phenylalanine 1-methyl ester, ammonium glycyrrhizate, salts thereof, and combinations thereof.

The term "sweetener" as used herein refers to any compound that is used to sweeten other products. Sweeteners can include sugars, sugar alcohols, monosaccharides, disaccharides, trisaccharides, oligosaccharides, sugar substitutes or mixtures thereof. Suitable sweeteners can include, but are not limited to, at least one of sucrose (saccharose), fructose, glucose, erythritol, maltitol, lactitol, sorbitol, mannitol, xylitol, D-tagatose, trehalose, galactose, rhamnose, cyclodextrin, ribulose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, glucosamine, mannosamine, fucose, glucuronic acid, gluconic acid, glucono-lactone, abequose, galactosamine, xylo-oligosaccharides, gentio-oligoscaccharides, galacto-oligosaccharides, sorbose, nigero-oligosaccharides, fructooligosaccharides, maltotetraol, maltotriol, malto-oligosaccharides, lactulose, melibiose, raffinose, rhamnose, ribose, isomerized liquid sugars such as high fructose corn/starch syrup, coupling sugars, soybean oligosaccharides, glucose syrup and mixtures thereof.

In a further embodiment of the pharmaceutical composition as herein disclosed, the pH modifier agent is sodium phosphate. Preferably, the pH modifier agent is di-sodium hydrogen phosphate, monosodium dihydrogen phosphate or mixtures thereof.

In a further embodiment of the pharmaceutical composition as herein disclosed, the total amount of pH modifier agent or pH modifier agents, preferably di-sodium hydrogen phosphate, monosodium dihydrogen phosphate and mixtures thereof, is below 8% w/w, preferably ranges from 0.1 to 5% w/w, more preferably from 1 to 3% w/w in respect of the total amount of the pharmaceutical composition.

The term "pH modifier" as used herein, means a compound that is capable of changing the pH of a solution. Examples of pH modifiers include, but are not limited to NaOH, LiOH, KOH, Na2CO3, NaHCO3, K2CO3, KHCO3, NaH2PO4, Na2HPO4, Na3PO4, KH2PO4, K2HPO4, K3PO4, meglumine, Ca(OH)2, Mg(OH)2, Zn(OH)2, Al(OH)3, pyridoxine, triethanolamine, ammonium hydroxide, cytosine, diethylamine, ornithine, glycine, lysine, arginine, valine, proline, aspartic acid, alanine, asparagine, isoleucine, leucine, methionine, threonine, choline hydroxide, procaine, diethylethanolamine, glucosamine, guanine, nicotinamide, piperazine, guanidine, olamine, piperidine, triethylamine, tromethamine, benzathine, benzathine, adenine, and mixtures thereof. Many of these compounds are well-known buffering agents. As used herein, the term "buffering agent" refers to any inorganic or organic acid or base that resists changes in pH and maintains the pH around a desired point.

The pharmaceutical compositions as herein disclosed are stable dosage forms. As used herein, the term "stable" refers to a pharmaceutical composition comprising dexketoprofen trometamol wherein the total content of impurities originating from the decomposition of dexketoprofen trometamol does not exceed 5% area, preferably 4% area, more preferably 3% area and most preferably 2% area determined by liquid chromatography at 233 nm if such a composition is stored for 3 months at 40 °C / 75% relative humidity (RH). Therefore, another aspect of the present invention relates to a pharmaceutical composition in the form of stick-pack comprising dexketoprofen trometamol, wherein the pharmaceutical composition comprises at least the following two portions: i) a filling solution, wherein the filling solution comprising dexketoprofen trometamol and at least a pharmaceutically acceptable excipient or excipients, wherein the dexketoprofen trometamol is dissolved in the filling solution and the pH of the filling solution is lower than 9; ii) a stick package, wherein the stick package comprises at least a sealed laminated foil of aluminium less than 20.0 microns thick; wherein the total content of impurities originating from the decomposition of dexketoprofen trometamol does not exceed 5% area, preferably 4% area, more preferably 3% area and most preferably 2% area determined by liquid chromatography if such a composition is stored for 3 months at 40 °C / 75% RH.

One advantage of the process to manufacture the pharmaceutical composition as herein disclosed is that does not require any special conditions or facilities to be carried out. The process as herein disclosed provides suitable solutions to be administered orally. Another advantage of the process to manufacture the pharmaceutical composition as herein disclosed is that the process is reproducible and uses non-toxic solvents resulting in an environmentally friendly process which can be performed in safety conditions by any technician.

In a further embodiment of the process to manufacture the pharmaceutical composition as herein disclosed, the pH of the filling solution in the step (g) is about 3 to 7.5. Preferably, the pH of the filling solution in the step (g) is about 4.0 to 7.0 and more preferably is about 5.7 to 6.5.

In a further embodiment of the process to manufacture the pharmaceutical composition as herein disclosed, the step (c) is keeping or, if necessary, cooling the temperature of the first mixture in step (a) or of the diluted first mixture in step (b) below 45 °C. Preferably, the temperature in step (c) is below 35 °C.

In a further embodiment of the process to manufacture the pharmaceutical composition as herein disclosed, the temperature of the first mixture in step (a) is between 43 and 60 °C. Preferably, the temperature of the first mixture in step (a) is between 43 and 60 °C and the temperature of the mixture in step (c) is below 35 °C.

In a further embodiment of the process to manufacture the pharmaceutical composition as herein disclosed, the temperature of the filling solution in step (g) is from 17 to 27 °C during the packaging step.

In a further embodiment of the process to manufacture the pharmaceutical composition as herein disclosed, the process comprises at least the following steps:
a) having a first mixture of polyethylene glycol 400, methyl paraben, neohesperidin dihydrochalcone and ammonium glycyrrhizate, and water at a temperature higher than 35 °C, preferably between 36 and 70 °C;
b) optionally diluting the first mixture with water;
c) preferably keeping or, if necessary, cooling the temperature of the first mixture prepared in step (a) or of the diluted first mixture prepared in step (b) below 45 °C;
d) adding dexketoprofen trometamol, saccharose, sodium saccharin, sodium dihydrogen phosphate dihydrate, di-sodium hydrogen phosphate anydrous, povidone and lemon natural flavour to the mixture of step (a), (b) or (c);
e) stirring the mixture obtained in the preceding step until a filling solution is obtained;
f) optionally, measuring the pH of the solution, and adjusting to a pH between 5.7 to 6.5 if required;
g) packaging the filling solution in a stick package, wherein the stick package comprises at least a sealed laminated foil of aluminium of less than 20.0 microns thick, preferably the temperature of the filling is from 17 to 27 °C during the packaging step.

In a further embodiment of the process to manufacture the pharmaceutical composition as herein disclosed, the process comprises at least the following steps:
a) having a first mixture of polyethylene glycol 400, methyl paraben, neohesperidin dihydrochalcone and ammonium glycyrrhizate, and water at a temperature higher than 35 °C, preferably between 36 and 70 °C;
b) diluting the first mixture with water;
c) keeping or, if necessary, cooling the temperature of the first mixture prepared in step (a) or of the diluted first mixture prepared in step (b) below 45 °C;
d) adding dexketoprofen trometamol, saccharose, sodium saccharin, sodium dihydrogen phosphate dihydrate, di-sodium hydrogen phosphate anydrous, povidone and lemon natural flavour to the mixture of step (a), (b) or (c);
e) stirring the mixture obtained in the preceding step until a filling solution is obtained;
f) measuring the pH of the solution, and adjusting to a pH between 5.7 to 6.5 if required;
g) packaging the filling solution in a stick package, wherein the stick package comprises at least a sealed laminated foil of aluminium of less than 20.0 microns thick and the temperature of the filling solution is from 17 to 27 °C during the packaging step.

In a further embodiment of the process to manufacture the pharmaceutical composition as herein disclosed, at least 15,000 units of pharmaceutical composition are manufactured.

In a further embodiment of the filling solution comprising dexketoprofen trometamol as herein disclosed, the filling solution is packaged in a stick package as defined in the above portion (ii).

This filling solution is pre-eminently suitable for filling into tightly closing sachets or stick packs.

The term "stick package" as used herein refers to the envelope of the stick-pack where the solution is filled in.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

Each pharmaceutical batch of finished product must fulfil the regulatory requirements of the corresponding Medicine Agency before being released for sale or supply, such as impurities thresholds and stability data.

The pharmaceutical composition may be manufactured at laboratory scale, not necessarily following GMP and not intended for commercialization. The pharmaceutical composition may also be manufactured for validation, following GMP. A batch of a pharmaceutical composition which is manufactured for validation is called "pilot batch".

In a further embodiment of the pharmaceutical batch as herein disclosed, is of at least 15,000 units of the pharmaceutical composition.

In a further embodiment of the pharmaceutical batch as herein disclosed, the relative standard deviation value of content uniformity is less than 5% w/w, preferably less than 1 % w/w.

Unless otherwise indicated, all the analysis methods are carried out according to the European Pharmacopoeia 7th edition.

Further aspects and embodiments of the present invention can be found in the following numbered clauses:
Clause 1.- A pharmaceutical composition in the form of stick-pack comprising dexketoprofen trometamol, wherein the pharmaceutical composition comprises at least the following two portions:
   i) a filling solution, wherein the filling solution comprises dexketoprofen trometamol and at least a pharmaceutically acceptable excipient or excipients, wherein the dexketoprofen trometamol is dissolved in the filling solution and the pH of the filling solution is lower than 9;
   ii) a stick package, wherein the stick package comprises at least a sealed laminated foil of aluminium of less than 20.0 microns thick.
Clause 2.- The pharmaceutical composition according to the preceding clause, wherein the sealed laminated foil of aluminium of the stick package is from 5 to 14.0 microns thick.
Clause 3.- The pharmaceutical composition according to any one of the preceding clauses, wherein the sealed laminated foil of aluminium of the stick package is from 7 to 12.0 microns thick.
Clause 4.- The pharmaceutical composition according to any one of the preceding clauses, wherein the sealed laminated sheet of aluminium of the stick package is less than 11.0 microns thick.
Clause 5.- The pharmaceutical composition according to any one of the preceding clauses, wherein the sealed laminated sheet of aluminium of the stick package is less than 10.0 microns thick.
Clause 6.- The pharmaceutical composition according to any one of the preceding clauses, wherein the sealed laminated sheet of aluminium of the stick package is about 9 microns thick.
Clause 7.- The pharmaceutical composition according to any one of the preceding clauses, wherein the pH of the filling solution is about 3 to 8.
Clause 8.- The pharmaceutical composition according to any one of the preceding clauses, wherein the pH of the filling solution is about 4.0 to 7.5.
Clause 9.- The pharmaceutical composition according to any one of the preceding clauses, wherein the pH of the filling solution is about 5.5 to 6.5.
Clause 10.- The pharmaceutical composition according to any one of the preceding clauses, wherein the stick package comprises at least an internal layer.
Clause 11.- The pharmaceutical composition according to the preceding clause, wherein the internal layer is of polyethylene.
Clause 12.- The pharmaceutical composition according to any one of the preceding clauses, wherein the stick package comprises at least an external layer.
Clause 13.- The pharmaceutical composition according to the preceding clause, wherein the external layer is of polyethylene.
Clause 14.- The pharmaceutical composition according to any one of the preceding clauses, wherein the stick package comprises at least an external layer and at least an internal layer.
Clause 15.- The pharmaceutical composition according to the preceding clause, wherein the stick package comprises at least a polyethylene external layer and at least a polyethylene internal layer.
Clause 16.- The pharmaceutical composition according to any one of the preceding clauses, wherein the stick package comprises at least an outer layer.
Clause 17.- The pharmaceutical composition according to the preceding clause, wherein the outer layer is printed paper or polyethylene terephthalate.
Clause 18.- The pharmaceutical composition according to the preceding clauses, wherein the stick package consists essentially of printed paper foil/polyethylene foil/aluminium foil/polyethylene foil or polyethylene terephthalate foil/polyethylene foil/aluminium foil/polyethylene foil.
Clause 19.-The pharmaceutical composition according to any one of the preceding clauses, wherein the concentration of dexketoprofen trometamol is about 0.5 to 10 mg/ml.
Clause 20.-The pharmaceutical composition according to any one of the preceding clauses, wherein the concentration of dexketoprofen trometamol is about 1 to 5 mg/ml.
Clause 21.- The pharmaceutical composition according to any one of the preceding clauses, wherein the concentration of dexketoprofen trometamol is about 2.5 mg/ml.
Clause 22.- The pharmaceutical composition according to any one of the preceding clauses, wherein the volume of the filling solution is from about 5 to 20 ml.
Clause 23.- The pharmaceutical composition according to the preceding clause, wherein the volume of the filling solution is from 7 to 15 ml.
Clause 24.- The pharmaceutical composition according to any one of the two preceding clauses, wherein the volume of the filling solution is from 9 to 12 ml.
Clause 25.- The pharmaceutical composition according to any one of the three preceding clauses, wherein the volume of the filling solution is about 10 ml.
Clause 26.- The pharmaceutical composition according to any one of the preceding clauses, comprising at least a viscosity agent, a preservative, a sweetness enhancer and, if required a pH modifier agent.
Clause 27.- The pharmaceutical composition according to the preceding clause, wherein the viscosity agent is selected from povidone, polyethylene glycol, and mixtures thereof.
Clause 28.- The pharmaceutical composition according to any of the two preceding clauses, wherein the total amount of amount of viscosity agent or viscosity agents, preferably povidone, polyethylene glycol and mixtures thereof, is below 60% w/w, preferably ranges from 10 to 50% w/w, more preferably from 15 to 45% w/w in respect of the total amount of the pharmaceutical composition.
Clause 29.- The pharmaceutical composition according to any of the three preceding clauses, wherein the amount of povidone is below 40% w/w, preferably ranges from 5 to 35% w/w, more preferably from 12 to 25% w/w in respect of the total amount of the pharmaceutical composition.
Clause 30.- The pharmaceutical composition according to any of the four preceding clauses, wherein the amount of polyethylene glycol is below 40% w/w, preferably ranges from 5 to 35% w/w, more preferably from 15 to 30% w/w in respect of the total amount of the pharmaceutical composition.
Clause 31.- The pharmaceutical composition according to any one of the five preceding clauses, wherein_the preservative is selected from salicylic acid, benzoic acid, methyl paraben, ethyl paraben, cresol, cetrimide, citric acid, EDTA and mixtures thereof.
Clause 32.- The pharmaceutical composition according to the preceding clauses, wherein the preservative agent is methyl paraben.
Clause 33.- The pharmaceutical composition according to any one of the two preceding clauses, wherein the amount of preservative agent, preferably methyl paraben, is_below 4% w/w, preferably ranges from 0.2 to 2% w/w, more preferably from 0.4 to 1% w/w in respect of the total amount of the pharmaceutical composition.
Clause 34.- The pharmaceutical composition according to any one of the eight preceding clauses, wherein the sweetness enhancer is selected from sucralose, acesulfame potassium or other salts, aspartame, alitame, sodium saccharin or other salts, neohesperidin dihydrochalcone, cyclamate, neotame, N-[3-(3-hydroxy-4-methoxyphenyl)propyl]-L-alpha.-aspartyl]-L-phenylalanine 1-methyl ester, N-[3-(3-hydroxy-4-methoxyphenyl)-3-methylbutyl]-L-alpha.-aspartyl]-phenylalanine 1-methyl ester, N-[3-(3-methoxy-4-hydroxyphenyl)propyl]-L-.alpha.-aspartyl]-L-phenylalanine 1-methyl ester, ammonium glycyrrhizate and mixtures thereof.
Clause 35.- The pharmaceutical composition according to the preceding clause, wherein_the sweetness enhancer is neohesperidin dihydrochalcone, sodium saccharin, ammonium glycyrrhizate or mixtures thereof.
Clause 36.- The pharmaceutical composition according to any one of the two preceding clauses, wherein the total amount of sweetness enhancer or sweetness enhancers, preferably neohesperidin dihydrochalcone, sodium saccharin, ammonium glycyrrhizate and mixtures thereof, is_below 2% w/w, preferably below 1% w/w, more preferably below 0.3% w/w in respect of the total amount of the pharmaceutical composition.
Clause 37.- The pharmaceutical composition according to any one of the eleven preceding clauses, wherein the pH modifier agent is sodium phosphate.
Clause 38.- The pharmaceutical composition according to the preceding clause, wherein the pH modifier agent is di-sodium hydrogen phosphate, monosodium dihydrogen phosphate or mixtures thereof.
Clause 39.- The pharmaceutical composition according to any one of the two preceding clauses, wherein the total amount of pH modifier agent or pH modifier agents, preferably di-sodium hydrogen phosphate, monosodium dihydrogen phosphate and mixtures thereof, is below 8% w/w, preferably ranges from 0.1 to 5% w/w, more preferably from 1 to 3% w/w in respect of the total amount of the pharmaceutical composition.
Clause 40.- A process for the manufacture of an pharmaceutical composition according to any one of the preceding clauses, wherein the process comprises at least the following steps:
   a) having a first mixture of at least a pharmaceutically acceptable excipient or excipients, and water at a temperature higher than 35 °C, preferably between 36 and 75 °C, wherein the pharmaceutically acceptable excipient or excipients being preferably selected from viscosity agents, preservatives, sweetness enhancers and mixtures thereof;
   b) optionally, diluting the first mixture with water;
   c) preferably keeping or, if necessary, cooling the temperature of the first mixture in step (a) or of the diluted mixture in step (b) below 45 °C;
   d) adding dexketoprofen trometamol, or a mixture of dexketoprofen trometamol and a further pharmaceutically acceptable excipient or excipients, to the mixture of step (a), (b) or (c);
   e) stirring the mixture obtained in the preceding step until a filling solution is obtained;
   f) optionally, measuring the pH of the filling solution, and adjusting to a pH below 9 if required;
   g) packaging the filling solution in a stick package, wherein the stick package comprises at least a sealed laminated foil of aluminium of less than 20.0 microns thick, preferably the temperature of the filling solution is at room temperature.
Clause 41.- The process for the manufacture of a pharmaceutical composition according to the preceding process clause, wherein the pH of the filling solution in the step (g) is about 3 to 7.5.
Clause 42.- The process for the manufacture of a pharmaceutical composition according to any one of the preceding process clauses, wherein the pH of the filling solution in the step (g) is about 4.0 to 7.0.
Clause 43.- The process for the manufacture of a pharmaceutical composition according to any one of the preceding process clauses, wherein the pH of the filling solution in the step (g) is about 5.7 to 6.5.
Clause 44.- The process for the manufacture of a pharmaceutical composition according to any one of the preceding process clauses, wherein_the step (c) is keeping or, if necessary, cooling the temperature of the first mixture in step (a) or of the diluted first mixture in step (b) below 45 °C.
Clause 45.- The process for the manufacture of a pharmaceutical composition according to any one of the preceding process clauses, wherein the temperature in step (c) is below 35 °C.
Clause 46.- The process for the manufacture of a pharmaceutical composition according to any one of the preceding process clauses, wherein the temperature of the first mixture in step (a) is between 43 and 60 °C.
Clause 47.- The process for the manufacture of a pharmaceutical composition according to any one of the preceding process clauses, wherein the temperature of the first mixture in step (a) is between 43 and 60 °C and the temperature of the mixture in step (c) is below 35 °C.
Clause 48.- The process for the manufacture of a pharmaceutical composition according to any one of the preceding process clauses, wherein the temperature of the filling solution in step (g) is from 17 to 27 °C during the packaging step.
Clause 49.- The process for the manufacture of a pharmaceutical composition according to any one of the preceding process clauses, wherein the process comprises at least the following steps:
   a) having a first mixture of polyethylene glycol 400, methyl paraben, neohesperidin dihydrochalcone and ammonium glycyrrhizate, and water at a temperature higher than 35 °C, preferably between 36 and 70 °C;
   b) optionally diluting the first mixture with water;
   c) preferably keeping or, if necessary, cooling the temperature of the first mixture prepared in step (a) or of the diluted first mixture prepared in step (b) below 45 °C;
   d) adding dexketoprofen trometamol, saccharose, sodium saccharin, sodium dihydrogen phosphate dihydrate, di-sodium hydrogen phosphate anhydrous, povidone and lemon natural flavour to the mixture of step (a), (b) or (c);
   e) stirring the mixture obtained in the preceding step until a filling solution is obtained;
   f) optionally, measuring the pH of the solution, and adjusting to a pH between 5.7 to 6.5 if required;
   g) packaging the filling solution in a stick package, wherein the stick package comprises at least a sealed laminated foil of aluminium of less than 20.0 microns thick, preferably the temperature of the filling solution is from 17 to 27 °C during the packaging step.
Clause 50.- The process according to any one of the preceding process clauses, wherein at least 15,000 units of pharmaceutical composition are manufactured.
Clause 51.- A filling solution comprising dexketoprofen trometamol, wherein the dexketoprofen trometamol is dissolved and the pH of the filling solution is lower than 9; wherein the filling solution is obtained by the process as defined in the steps of any of the preceding process clauses.
Clause 52.- The filling solution according to previous clause, wherein the filling solution is packaged in a stick package as defined in the above portion (ii) of the preceding clauses.
Clause 53.- A pharmaceutical batch of at least 15,000 units of the pharmaceutical composition according to any one of the preceding product clauses.
Clause 54.- The pharmaceutical batch according to the preceding clause, wherein the relative standard deviation value of content uniformity is less than 5% w/w, preferably less than 1 % w/w.
Clause 55.- A cardboard box with patient information leaflet in pack size of at least 4 stick -pack as defined in any one of the preceding product clauses.
Clause 56.- A pharmaceutical composition in the form of stick-pack comprising dexketoprofen trometamol, wherein the pharmaceutical composition comprises at least the following two portions:
   i) a filling solution, wherein the filling solution comprises dexketoprofen trometamol and at least a pharmaceutically acceptable excipient or excipients, wherein the dexketoprofen trometamol is dissolved in the filling solution and the pH of the filling solution is lower than 9;
   ii) a stick package, wherein the stick package comprises at least a sealed laminated foil of aluminium of less than 20.0 microns thick.
   wherein the pharmaceutical composition is manufactured by the process comprising at least the following steps:
   a) having a first mixture of at least a pharmaceutically acceptable excipient or excipients, and water at a temperature higher than 35 °C, preferably between 36 and 75 °C, more preferably between 43 and 60 °C, wherein the pharmaceutically acceptable excipient or excipients being preferably selected from viscosity agents, preservatives, sweetness enhancers and mixtures thereof;
   b) optionally, diluting the first mixture with water;
   c) preferably keeping or, if necessary, cooling the temperature of the first mixture in step (a) or of the diluted mixture in step (b) below 45 °C, more preferably below 35 °C,
   d) adding dexketoprofen trometamol, or a mixture of dexketoprofen trometamol and a further pharmaceutically acceptable excipient or excipients, to the mixture of step (a), (b) or (c);
   e) stirring the mixture obtained in the preceding step;
   f) optionally, measuring the pH of the filling solution, and adjusting to a pH below 9 if required;
   g) packaging the filling solution in a stick package, wherein the stick package comprises at least a sealed laminated foil of aluminium of less than 20.0 microns thick;
   optionally, at least 15,000 units of pharmaceutical composition are manufactured.

Preferred combinations of the above clauses are as follows:

| | |
|---|---|
| Clause 57.- 1, 3 | Clause 345.- 1, 3, 39 |
| Clause 58.- 1, 5 | Clause 346.- 1, 5, 39 |
| Clause 59.- 1, 8 | Clause 347.- 1, 8, 39 |
| Clause 60.- 1, 3, 8 | Clause 348.- 1, 3, 8, 39 |
| Clause 61.- 1, 5, 8 | Clause 349.- 1, 5, 8, 39 |
| Clause 62.- 1, 15 | Clause 350.- 1, 15, 39 |
| Clause 63.- 1, 3, 15 | Clause 351.- 1, 3, 15, 39 |
| Clause 64.- 1, 5, 15 | Clause 352.- 1, 5, 15, 39 |
| Clause 65.- 1, 8, 15 | Clause 353.- 1, 8, 15, 39 |
| Clause 66.- 1, 3, 8, 15 | Clause 354.- 1, 3, 8, 15, 39 |
| Clause 67.- 1, 5, 8, 15 | Clause 355.- 1, 5, 8, 15, 39 |
| Clause 68.- 1, 21 | Clause 356.- 1, 21, 39 |
| Clause 69.- 1, 3, 21 | Clause 357.- 1, 3, 21, 39 |
| Clause 70.- 1, 5, 21 | Clause 358.- 1, 5, 21, 39 |
| Clause 71.- 1, 8, 21 | Clause 359.- 1, 8, 21, 39 |
| Clause 72.- 1, 3, 8, 21 | Clause 360.- 1, 3, 8, 21, 39 |
| Clause 73.- 1, 5, 8, 21 | Clause 361.- 1, 5, 8, 21, 39 |
| Clause 74.- 1, 15, 21 | Clause 362.- 1, 15, 21, 39 |
| Clause 75.- 1, 3, 15, 21 | Clause 363.- 1, 3, 15, 21, 39 |
| Clause 76.- 1, 5, 15, 21 | Clause 364.- 1, 5, 15, 21, 39 |
| Clause 77.- 1, 8, 15, 21 | Clause 365.- 1, 8, 15, 21, 39 |
| Clause 78.- 1, 3, 8, 15, 21 | Clause 366.- 1, 3, 8, 15, 21, 39 |
| Clause 79.- 1, 5, 8, 15, 21 | Clause 367.- 1, 5, 8, 15, 21, 39 |
| Clause 80.- 1, 25 | Clause 368.- 1, 25, 39 |
| Clause 81.- 1, 3, 25 | Clause 369.- 1, 3, 25, 39 |
| Clause 82.- 1, 5, 25 | Clause 370.- 1, 5, 25, 39 |
| Clause 83.- 1, 8, 25 | Clause 371.- 1, 8, 25, 39 |
| Clause 84.- 1, 3, 8, 25 | Clause 372.- 1, 3, 8, 25, 39 |
| Clause 85.- 1, 5, 8, 25 | Clause 373.- 1, 5, 8, 25, 39 |
| Clause 86.- 1, 15, 25 | Clause 374.- 1, 15, 25, 39 |
| Clause 87.- 1, 3, 15, 25 | Clause 375.- 1, 3, 15, 25, 39 |
| Clause 88.- 1, 5, 15, 25 | Clause 376.- 1, 5, 15, 25, 39 |
| Clause 89.- 1, 8, 15, 25 | Clause 377.- 1, 8, 15, 25, 39 |
| Clause 90.- 1, 3, 8, 15, 25 | Clause 378.- 1, 3, 8, 15, 25, 39 |
| Clause 91.- 1, 5, 8, 15, 25 | Clause 379.- 1, 5, 8, 15, 25, 39 |
| Clause 92.- 1, 21, 25 | Clause 380.- 1, 21, 25, 39 |
| Clause 93.- 1, 3, 21, 25 | Clause 381.- 1, 3, 21, 25, 39 |
| Clause 94.- 1, 5, 21, 25 | Clause 382.- 1, 5, 21, 25, 39 |
| Clause 95.- 1, 8, 21, 25 | Clause 383.- 1, 8, 21, 25, 39 |
| Clause 96.- 1, 3, 8, 21, 25 | Clause 384.- 1, 3, 8, 21, 25, 39 |
| Clause 97.- 1, 5, 8, 21, 25 | Clause 385.- 1, 5, 8, 21, 25, 39 |
| Clause 98.- 1, 15, 21, 25 | Clause 386.- 1, 15, 21, 25, 39 |
| Clause 99.- 1, 3, 15, 21, 25 | Clause 387.- 1, 3, 15, 21, 25, 39 |
| Clause 100.- 1, 5, 15, 21, 25 | Clause 388.- 1, 5, 15, 21, 25, 39 |
| Clause 101.- 1, 8, 15, 21, 25 | Clause 389.- 1, 8, 15, 21, 25, 39 |
| Clause 102.- 1, 3, 8, 15, 21, 25 | Clause 390.- 1, 3, 8, 15, 21, 25, 39 |
| Clause 103.- 1, 5, 8, 15, 21, 25 | Clause 391.- 1, 5, 8, 15, 21, 25, 39 |
| Clause 104.- 1, 28 | Clause 392.- 1, 28, 39 |
| Clause 105.- 1, 3, 28 | Clause 393.- 1, 3, 28, 39 |
| Clause 106.- 1, 5, 28 | Clause 394.- 1, 5, 28, 39 |
| Clause 107.- 1, 8, 28 | Clause 395.- 1, 8, 28, 39 |
| Clause 108.- 1, 3, 8, 28 | Clause 396.- 1, 3, 8, 28, 39 |
| Clause 109.- 1, 5, 8, 28 | Clause 397.- 1, 5, 8, 28, 39 |
| Clause 110.- 1, 15, 28 | Clause 398.- 1, 15, 28, 39 |
| Clause 111.- 1, 3, 15, 28 | Clause 399.- 1, 3, 15, 28, 39 |
| Clause 112.- 1, 5, 15, 28 | Clause 400.- 1, 5, 15, 28, 39 |
| Clause 113.- 1, 8, 15, 28 | Clause 401.- 1, 8, 15, 28, 39 |
| Clause 114.- 1, 3, 8, 15, 28 | Clause 402.- 1, 3, 8, 15, 28, 39 |
| Clause 115.- 1, 5, 8, 15, 28 | Clause 403.- 1, 5, 8, 15, 28, 39 |
| Clause 116.- 1, 21, 28 | Clause 404.- 1, 21, 28, 39 |
| Clause 117.- 1, 3, 21, 28 | Clause 405.- 1, 3, 21, 28, 39 |
| Clause 118.- 1, 5, 21, 28 | Clause 406.- 1, 5, 21, 28, 39 |
| Clause 119.- 1, 8, 21, 28 | Clause 407.- 1, 8, 21, 28, 39 |
| Clause 120.- 1, 3, 8, 21, 28 | Clause 408.- 1, 3, 8, 21, 28, 39 |
| Clause 121.- 1, 5, 8, 21, 28 | Clause 409.- 1, 5, 8, 21, 28, 39 |
| Clause 122.- 1, 15, 21, 28 | Clause 410.- 1, 15, 21, 28, 39 |
| Clause 123.- 1, 3, 15, 21, 28 | Clause 411.- 1, 3, 15, 21, 28, 39 |
| Clause 124.- 1, 5, 15, 21, 28 | Clause 412.- 1, 5, 15, 21, 28, 39 |
| Clause 125.- 1, 8, 15, 21, 28 | Clause 413.- 1, 8, 15, 21, 28, 39 |
| Clause 126.- 1, 3, 8, 15, 21, 28 | Clause 414.- 1, 3, 8, 15, 21, 28, 39 |
| Clause 127.- 1, 5, 8, 15, 21, 28 | Clause 415.- 1, 5, 8, 15, 21, 28, 39 |
| Clause 128.- 1, 25, 28 | Clause 416.- 1, 25, 28, 39 |
| Clause 129.- 1, 3, 25, 28 | Clause 417.- 1, 3, 25, 28, 39 |
| Clause 130.- 1, 5, 25, 28 | Clause 418.- 1, 5, 25, 28, 39 |
| Clause 131.- 1, 8, 25, 28 | Clause 419.- 1, 8, 25, 28, 39 |
| Clause 132.- 1, 3, 8, 25, 28 | Clause 420.- 1, 3, 8, 25, 28, 39 |
| Clause 133.- 1, 5, 8, 25, 28 | Clause 421.- 1, 5, 8, 25, 28, 39 |
| Clause 134.- 1, 15, 25, 28 | Clause 422.- 1, 15, 25, 28, 39 |
| Clause 135.- 1, 3, 15, 25, 28 | Clause 423.- 1, 3, 15, 25, 28, 39 |
| Clause 136.- 1, 5, 15, 25, 28 | Clause 424.- 1, 5, 15, 25, 28, 39 |
| Clause 137.- 1, 8, 15, 25, 28 | Clause 425.- 1, 8, 15, 25, 28, 39 |
| Clause 138.- 1, 3, 8, 15, 25, 28 | Clause 426.- 1, 3, 8, 15, 25, 28, 39 |
| Clause 139.- 1, 5, 8, 15, 25, 28 | Clause 427.- 1, 5, 8, 15, 25, 28, 39 |
| Clause 140.- 1, 21, 25, 28 | Clause 428.- 1, 21, 25, 28, 39 |
| Clause 141.- 1, 3, 21, 25, 28 | Clause 429.- 1, 3, 21, 25, 28, 39 |
| Clause 142.- 1, 5, 21, 25, 28 | Clause 430.- 1, 5, 21, 25, 28, 39 |
| Clause 143.- 1, 8, 21, 25, 28 | Clause 431.- 1, 8, 21, 25, 28, 39 |
| Clause 144.- 1, 3, 8, 21, 25, 28 | Clause 432.- 1, 3, 8, 21, 25, 28, 39 |
| Clause 145.- 1, 5, 8, 21, 25, 28 | Clause 433.- 1, 5, 8, 21, 25, 28, 39 |
| Clause 146.- 1, 15, 21, 25, 28 | Clause 434.- 1, 15, 21, 25, 28, 39 |
| Clause 147.- 1, 3, 15, 21, 25, 28 | Clause 435.- 1, 3, 15, 21, 25, 28, 39 |
| Clause 148.- 1, 5, 15, 21, 25, 28 | Clause 436.- 1, 5, 15, 21, 25, 28, 39 |
| Clause 149.- 1, 8, 15, 21, 25, 28 | Clause 437.- 1, 8, 15, 21, 25, 28, 39 |
| Clause 150.- 1, 3, 8, 15, 21, 25, 28 | Clause 438.- 1, 3, 8, 15, 21, 25, 28, 39 |
| Clause 151.- 1, 5, 8, 15, 21, 25, 28 | Clause 439.- 1, 5, 8, 15, 21, 25, 28, 39 |
| Clause 152.- 1, 33 | Clause 440.- 1, 33, 39 |
| Clause 153.- 1, 3, 33 | Clause 441.- 1, 3, 33, 39 |
| Clause 154.- 1, 5, 33 | Clause 442.- 1, 5, 33, 39 |
| Clause 155.- 1, 8, 33 | Clause 443.- 1, 8, 33, 39 |
| Clause 156.- 1, 3, 8, 33 | Clause 444.- 1, 3, 8, 33, 39 |
| Clause 157.- 1, 5, 8, 33 | Clause 445.- 1, 5, 8, 33, 39 |
| Clause 158.- 1, 15, 33 | Clause 446.- 1, 15, 33, 39 |
| Clause 159.- 1, 3, 15, 33 | Clause 447.- 1, 3, 15, 33, 39 |
| Clause 160.- 1, 5, 15, 33 | Clause 448.- 1, 5, 15, 33, 39 |
| Clause 161.- 1, 8, 15, 33 | Clause 449.- 1, 8, 15, 33, 39 |
| Clause 162.- 1, 3, 8, 15, 33 | Clause 450.- 1, 3, 8, 15, 33, 39 |
| Clause 163.- 1, 5, 8, 15, 33 | Clause 451.- 1, 5, 8, 15, 33, 39 |
| Clause 164.- 1, 21, 33 | Clause 452.- 1, 21, 33, 39 |
| Clause 165.- 1, 3, 21, 33 | Clause 453.- 1, 3, 21, 33, 39 |
| Clause 166.- 1, 5, 21, 33 | Clause 454.- 1, 5, 21, 33, 39 |
| Clause 167.- 1, 8, 21, 33 | Clause 455.- 1, 8, 21, 33, 39 |
| Clause 168.- 1, 3, 8, 21, 33 | Clause 456.- 1, 3, 8, 21, 33, 39 |
| Clause 169.- 1, 5, 8, 21, 33 | Clause 457.- 1, 5, 8, 21, 33, 39 |
| Clause 170.- 1, 15, 21, 33 | Clause 458.- 1, 15, 21, 33, 39 |
| Clause 171.- 1, 3, 15, 21, 33 | Clause 459.- 1, 3, 15, 21, 33, 39 |
| Clause 172.- 1, 5, 15, 21, 33 | Clause 460.- 1, 5, 15, 21, 33, 39 |
| Clause 173.- 1, 8, 15, 21, 33 | Clause 461.- 1, 8, 15, 21, 33, 39 |
| Clause 174.- 1, 3, 8, 15, 21, 33 | Clause 462.- 1, 3, 8, 15, 21, 33, 39 |
| Clause 175.- 1, 5, 8, 15, 21, 33 | Clause 463.- 1, 5, 8, 15, 21, 33, 39 |
| Clause 176.- 1, 25, 33 | Clause 464.- 1, 25, 33, 39 |
| Clause 177.- 1, 3, 25, 33 | Clause 465.- 1, 3, 25, 33, 39 |
| Clause 178.- 1, 5, 25, 33 | Clause 466.- 1, 5, 25, 33, 39 |
| Clause 179.- 1, 8, 25, 33 | Clause 467.- 1, 8, 25, 33, 39 |
| Clause 180.- 1, 3, 8, 25, 33 | Clause 468.- 1, 3, 8, 25, 33, 39 |
| Clause 181.- 1, 5, 8, 25, 33 | Clause 469.- 1, 5, 8, 25, 33, 39 |
| Clause 182.- 1, 15, 25, 33 | Clause 470.- 1, 15, 25, 33, 39 |
| Clause 183.- 1, 3, 15, 25, 33 | Clause 471.- 1, 3, 15, 25, 33, 39 |
| Clause 184.- 1, 5, 15, 25, 33 | Clause 472.- 1, 5, 15, 25, 33, 39 |
| Clause 185.- 1, 8, 15, 25, 33 | Clause 473.- 1, 8, 15, 25, 33, 39 |
| Clause 186.- 1, 3, 8, 15, 25, 33 | Clause 474.- 1, 3, 8, 15, 25, 33, 39 |
| Clause 187.- 1, 5, 8, 15, 25, 33 | Clause 475.- 1, 5, 8, 15, 25, 33, 39 |
| Clause 188.- 1, 21, 25, 33 | Clause 476.- 1, 21, 25, 33, 39 |
| Clause 189.- 1, 3, 21, 25, 33 | Clause 477.- 1, 3, 21, 25, 33, 39 |
| Clause 190.- 1, 5, 21, 25, 33 | Clause 478.- 1, 5, 21, 25, 33, 39 |
| Clause 191.- 1, 8, 21, 25, 33 | Clause 479.- 1, 8, 21, 25, 33, 39 |
| Clause 192.- 1, 3, 8, 21, 25, 33 | Clause 480.- 1, 3, 8, 21, 25, 33, 39 |
| Clause 193.- 1, 5, 8, 21, 25, 33 | Clause 481.- 1, 5, 8, 21, 25, 33, 39 |
| Clause 194.- 1, 15, 21, 25, 33 | Clause 482.- 1, 15, 21, 25, 33, 39 |
| Clause 195.- 1, 3, 15, 21, 25, 33 | Clause 483.- 1, 3, 15, 21, 25, 33, 39 |
| Clause 196.- 1, 5, 15, 21, 25, 33 | Clause 484.- 1, 5, 15, 21, 25, 33, 39 |
| Clause 197.- 1, 8, 15, 21, 25, 33 | Clause 485.- 1, 8, 15, 21, 25, 33, 39 |
| Clause 198.- 1, 3, 8, 15, 21, 25, 33 | Clause 486.- 1, 3, 8, 15, 21, 25, 33, 39 |
| Clause 199.- 1, 5, 8, 15, 21, 25, 33 | Clause 487.- 1, 5, 8, 15, 21, 25, 33, 39 |
| Clause 200.- 1, 28, 33 | Clause 488.- 1, 28, 33, 39 |
| Clause 201.- 1, 3, 28, 33 | Clause 489.- 1, 3, 28, 33, 39 |
| Clause 202.- 1, 5, 28, 33 | Clause 490.- 1, 5, 28, 33, 39 |
| Clause 203.- 1, 8, 28, 33 | Clause 491.- 1, 8, 28, 33, 39 |
| Clause 204.- 1, 3, 8, 28, 33 | Clause 492.- 1, 3, 8, 28, 33, 39 |
| Clause 205.- 1, 5, 8, 28, 33 | Clause 493.- 1, 5, 8, 28, 33, 39 |
| Clause 206.- 1, 15, 28, 33 | Clause 494.- 1, 15, 28, 33, 39 |
| Clause 207.- 1, 3, 15, 28, 33 | Clause 495.- 1, 3, 15, 28, 33, 39 |
| Clause 208.- 1, 5, 15, 28, 33 | Clause 496.- 1, 5, 15, 28, 33, 39 |
| Clause 209.- 1, 8, 15, 28, 33 | Clause 497.- 1, 8, 15, 28, 33, 39 |
| Clause 210.- 1, 3, 8, 15, 28, 33 | Clause 498.- 1, 3, 8, 15, 28, 33, 39 |
| Clause 211.- 1, 5, 8, 15, 28, 33 | Clause 499.- 1, 5, 8, 15, 28, 33, 39 |
| Clause 212.- 1, 21, 28, 33 | Clause 500.- 1, 21, 28, 33, 39 |
| Clause 213.- 1, 3, 21, 28, 33 | Clause 501.- 1, 3, 21, 28, 33, 39 |
| Clause 214.- 1, 5, 21, 28, 33 | Clause 502.- 1, 5, 21, 28, 33, 39 |
| Clause 215.- 1, 8, 21, 28, 33 | Clause 503.- 1, 8, 21, 28, 33, 39 |
| Clause 216.- 1, 3, 8, 21, 28, 33 | Clause 504.- 1, 3, 8, 21, 28, 33, 39 |
| Clause 217.- 1, 5, 8, 21, 28, 33 | Clause 505.- 1, 5, 8, 21, 28, 33, 39 |
| Clause 218.- 1, 15, 21, 28, 33 | Clause 506.- 1, 15, 21, 28, 33, 39 |
| Clause 219.- 1, 3, 15, 21, 28, 33 | Clause 507.- 1, 3, 15, 21, 28, 33, 39 |
| Clause 220.- 1, 5, 15, 21, 28, 33 | Clause 508.- 1, 5, 15, 21, 28, 33, 39 |
| Clause 221.- 1, 8, 15, 21, 28, 33 | Clause 509.- 1, 8, 15, 21, 28, 33, 39 |
| Clause 222.- 1, 3, 8, 15, 21, 28, 33 | Clause 510.- 1, 3, 8, 15, 21, 28, 33, 39 |
| Clause 223.- 1, 5, 8, 15, 21, 28, 33 | Clause 511.- 1, 5, 8, 15, 21, 28, 33, 39 |
| Clause 224.- 1, 25, 28, 33 | Clause 512.- 1, 25, 28, 33, 39 |
| Clause 225.- 1, 3, 25, 28, 33 | Clause 513.- 1, 3, 25, 28, 33, 39 |
| Clause 226.- 1, 5, 25, 28, 33 | Clause 514.- 1, 5, 25, 28, 33, 39 |
| Clause 227.- 1, 8, 25, 28, 33 | Clause 515.- 1, 8, 25, 28, 33, 39 |
| Clause 228.- 1, 3, 8, 25, 28, 33 | Clause 516.- 1, 3, 8, 25, 28, 33, 39 |
| Clause 229.- 1, 5, 8, 25, 28, 33 | Clause 517.- 1, 5, 8, 25, 28, 33, 39 |
| Clause 230.- 1, 15, 25, 28, 33 | Clause 518.- 1, 15, 25, 28, 33, 39 |
| Clause 231.- 1, 3, 15, 25, 28, 33 | Clause 519.- 1, 3, 15, 25, 28, 33, 39 |
| Clause 232.- 1, 5, 15, 25, 28, 33 | Clause 520.- 1, 5, 15, 25, 28, 33, 39 |
| Clause 233.- 1, 8, 15, 25, 28, 33 | Clause 521.- 1, 8, 15, 25, 28, 33, 39 |
| Clause 234.- 1, 3, 8, 15, 25, 28, 33 | Clause 522.- 1, 3, 8, 15, 25, 28, 33, 39 |
| Clause 235.- 1, 5, 8, 15, 25, 28, 33 | Clause 523.- 1, 5, 8, 15, 25, 28, 33, 39 |
| Clause 236.- 1, 21, 25, 28, 33 | Clause 524.- 1, 21, 25, 28, 33, 39 |
| Clause 237.- 1, 3, 21, 25, 28, 33 | Clause 525.- 1, 3, 21, 25, 28, 33, 39 |
| Clause 238.- 1, 5, 21, 25, 28, 33 | Clause 526.- 1, 5, 21, 25, 28, 33, 39 |
| Clause 239.- 1, 8, 21, 25, 28, 33 | Clause 527.- 1, 8, 21, 25, 28, 33, 39 |
| Clause 240.- 1, 3, 8, 21, 25, 28, 33 | Clause 528.- 1, 3, 8, 21, 25, 28, 33, 39 |
| Clause 241.- 1, 5, 8, 21, 25, 28, 33 | Clause 529.- 1, 5, 8, 21, 25, 28, 33, 39 |
| Clause 242.- 1, 15, 21, 25, 28, 33 | Clause 530.- 1, 15, 21, 25, 28, 33, 39 |
| Clause 243.- 1, 3, 15, 21, 25, 28, 33 | Clause 531.- 1, 3, 15, 21, 25, 28, 33, 39 |
| Clause 244.- 1, 5, 15, 21, 25, 28, 33 | Clause 532.- 1, 5, 15, 21, 25, 28, 33, 39 |
| Clause 245.- 1, 8, 15, 21, 25, 28, 33 | Clause 533.- 1, 8, 15, 21, 25, 28, 33, 39 |
| Clause 246.- 1, 3, 8, 15, 21, 25, 28, 33 | Clause 534.- 1, 3, 8, 15, 21, 25, 28, 33, 39 |
| Clause 247.- 1, 5, 8, 15, 21, 25, 28, 33 | Clause 535.- 1, 5, 8, 15, 21, 25, 28, 33, 39 |
| Clause 248.- 1, 36 | Clause 536.- 1, 36, 39 |
| Clause 249.- 1, 3, 36 | Clause 537.- 1, 3, 36, 39 |
| Clause 250.- 1, 5, 36 | Clause 538.- 1, 5, 36, 39 |
| Clause 251.- 1, 8, 36 | Clause 539.- 1, 8, 36, 39 |
| Clause 252.- 1, 3, 8, 36 | Clause 540.- 1, 3, 8, 36, 39 |
| Clause 253.- 1, 5, 8, 36 | Clause 541.- 1, 5, 8, 36, 39 |
| Clause 254.- 1, 15, 36 | Clause 542.- 1, 15, 36, 39 |
| Clause 255.- 1, 3, 15, 36 | Clause 543.- 1, 3, 15, 36, 39 |
| Clause 256.- 1, 5, 15, 36 | Clause 544.- 1, 5, 15, 36, 39 |
| Clause 257.- 1, 8, 15, 36 | Clause 545.- 1, 8, 15, 36, 39 |
| Clause 258.- 1, 3, 8, 15, 36 | Clause 546.- 1, 3, 8, 15, 36, 39 |
| Clause 259.- 1, 5, 8, 15, 36 | Clause 547.- 1, 5, 8, 15, 36, 39 |
| Clause 260.- 1, 21, 36 | Clause 548.- 1, 21, 36, 39 |
| Clause 261.- 1, 3, 21, 36 | Clause 549.- 1, 3, 21, 36, 39 |
| Clause 262.- 1, 5, 21, 36 | Clause 550.- 1, 5, 21, 36, 39 |
| Clause 263.- 1, 8, 21, 36 | Clause 551.- 1, 8, 21, 36, 39 |
| Clause 264.- 1, 3, 8, 21, 36 | Clause 552.- 1, 3, 8, 21, 36, 39 |
| Clause 265.- 1, 5, 8, 21, 36 | Clause 553.- 1, 5, 8, 21, 36, 39 |
| Clause 266.- 1, 15, 21, 36 | Clause 554.- 1, 15, 21, 36, 39 |
| Clause 267.- 1, 3, 15, 21, 36 | Clause 555.- 1, 3, 15, 21, 36, 39 |
| Clause 268.- 1, 5, 15, 21, 36 | Clause 556.- 1, 5, 15, 21, 36, 39 |
| Clause 269.- 1, 8, 15, 21, 36 | Clause 557.- 1, 8, 15, 21, 36, 39 |
| Clause 270.- 1, 3, 8, 15, 21, 36 | Clause 558.- 1, 3, 8, 15, 21, 36, 39 |
| Clause 271.- 1, 5, 8, 15, 21, 36 | Clause 559.- 1, 5, 8, 15, 21, 36, 39 |
| Clause 272.- 1, 25, 36 | Clause 560.- 1, 25, 36, 39 |
| Clause 273.- 1, 3, 25, 36 | Clause 561.- 1, 3, 25, 36, 39 |
| Clause 274.- 1, 5, 25, 36 | Clause 562.- 1, 5, 25, 36, 39 |
| Clause 275.- 1, 8, 25, 36 | Clause 563.- 1, 8, 25, 36, 39 |
| Clause 276.- 1, 3, 8, 25, 36 | Clause 564.- 1, 3, 8, 25, 36, 39 |
| Clause 277.- 1, 5, 8, 25, 36 | Clause 565.- 1, 5, 8, 25, 36, 39 |
| Clause 278.- 1, 15, 25, 36 | Clause 566.- 1, 15, 25, 36, 39 |
| Clause 279.- 1, 3, 15, 25, 36 | Clause 567.- 1, 3, 15, 25, 36, 39 |
| Clause 280.- 1, 5, 15, 25, 36 | Clause 568.- 1, 5, 15, 25, 36, 39 |
| Clause 281.- 1, 8, 15, 25, 36 | Clause 569.- 1, 8, 15, 25, 36, 39 |
| Clause 282.- 1, 3, 8, 15, 25, 36 | Clause 570.- 1, 3, 8, 15, 25, 36, 39 |
| Clause 283.- 1, 5, 8, 15, 25, 36 | Clause 571.- 1, 5, 8, 15, 25, 36, 39 |
| Clause 284.- 1, 21, 25, 36 | Clause 572.- 1, 21, 25, 36, 39 |
| Clause 285.- 1, 3, 21, 25, 36 | Clause 573.- 1, 3, 21, 25, 36, 39 |
| Clause 286.- 1, 5, 21, 25, 36 | Clause 574.- 1, 5, 21, 25, 36, 39 |
| Clause 287.- 1, 8, 21, 25, 36 | Clause 575.- 1, 8, 21, 25, 36, 39 |
| Clause 288.- 1, 3, 8, 21, 25, 36 | Clause 576.- 1, 3, 8, 21, 25, 36, 39 |
| Clause 289.- 1, 5, 8, 21, 25, 36 | Clause 577.- 1, 5, 8, 21, 25, 36, 39 |
| Clause 290.- 1, 15, 21, 25, 36 | Clause 578.- 1, 15, 21, 25, 36, 39 |
| Clause 291.- 1, 3, 15, 21, 25, 36 | Clause 579.- 1, 3, 15, 21, 25, 36, 39 |
| Clause 292.- 1, 5, 15, 21, 25, 36 | Clause 580.- 1, 5, 15, 21, 25, 36, 39 |
| Clause 293.- 1, 8, 15, 21, 25, 36 | Clause 581.- 1, 8, 15, 21, 25, 36, 39 |
| Clause 294.- 1, 3, 8, 15, 21, 25, 36 | Clause 582.- 1, 3, 8, 15, 21, 25, 36, 39 |
| Clause 295.- 1, 5, 8, 15, 21, 25, 36 | Clause 583.- 1, 5, 8, 15, 21, 25, 36, 39 |
| Clause 296.- 1, 28, 36 | Clause 584.- 1, 28, 36, 39 |
| Clause 297.- 1, 3, 28, 36 | Clause 585.- 1, 3, 28, 36, 39 |
| Clause 298.- 1, 5, 28, 36 | Clause 586.- 1, 5, 28, 36, 39 |
| Clause 299.- 1, 8, 28, 36 | Clause 587.- 1, 8, 28, 36, 39 |
| Clause 300.- 1, 3, 8, 28, 36 | Clause 588.- 1, 3, 8, 28, 36, 39 |
| Clause 301.- 1, 5, 8, 28, 36 | Clause 589.- 1, 5, 8, 28, 36, 39 |
| Clause 302.- 1, 15, 28, 36 | Clause 590.- 1, 15, 28, 36, 39 |
| Clause 303.- 1, 3, 15, 28, 36 | Clause 591.- 1, 3, 15, 28, 36, 39 |
| Clause 304.- 1, 5, 15, 28, 36 | Clause 592.- 1, 5, 15, 28, 36, 39 |
| Clause 305.- 1, 8, 15, 28, 36 | Clause 593.- 1, 8, 15, 28, 36, 39 |
| Clause 306.- 1, 3, 8, 15, 28, 36 | Clause 594.- 1, 3, 8, 15, 28, 36, 39 |
| Clause 307.- 1, 5, 8, 15, 28, 36 | Clause 595.- 1, 5, 8, 15, 28, 36, 39 |
| Clause 308.- 1, 21, 28, 36 | Clause 596.- 1, 21, 28, 36, 39 |
| Clause 309.- 1, 3, 21, 28, 36 | Clause 597.- 1, 3, 21, 28, 36, 39 |
| Clause 310.- 1, 5, 21, 28, 36 | Clause 598.- 1, 5, 21, 28, 36, 39 |
| Clause 311.- 1, 8, 21, 28, 36 | Clause 599.- 1, 8, 21, 28, 36, 39 |
| Clause 312.- 1, 3, 8, 21, 28, 36 | Clause 600.- 1, 3, 8, 21, 28, 36, 39 |
| Clause 313.- 1, 5, 8, 21, 28, 36 | Clause 601.- 1, 5, 8, 21, 28, 36, 39 |
| Clause 314.- 1, 15, 21, 28, 36 | Clause 602.- 1, 15, 21, 28, 36, 39 |
| Clause 315.- 1, 3, 15, 21, 28, 36 | Clause 603.- 1, 3, 15, 21, 28, 36, 39 |
| Clause 316.- 1, 5, 15, 21, 28, 36 | Clause 604.- 1, 5, 15, 21, 28, 36, 39 |
| Clause 317.- 1, 8, 15, 21, 28, 36 | Clause 605.- 1, 8, 15, 21, 28, 36, 39 |
| Clause 318.- 1, 3, 8, 15, 21, 28, 36 | Clause 606.- 1, 3, 8, 15, 21, 28, 36, 39 |
| Clause 319.- 1, 5, 8, 15, 21, 28, 36 | Clause 607.- 1, 5, 8, 15, 21, 28, 36, 39 |
| Clause 320.- 1, 25, 28, 36 | Clause 608.- 1, 25, 28, 36, 39 |
| Clause 321.- 1, 3, 25, 28, 36 | Clause 609.- 1, 3, 25, 28, 36, 39 |
| Clause 322.- 1, 5, 25, 28, 36 | Clause 610.- 1, 5, 25, 28, 36, 39 |
| Clause 323.- 1, 8, 25, 28, 36 | Clause 611.- 1, 8, 25, 28, 36, 39 |
| Clause 324.- 1, 3, 8, 25, 28, 36 | Clause 612.- 1, 3, 8, 25, 28, 36, 39 |
| Clause 325.- 1, 5, 8, 25, 28, 36 | Clause 613.- 1, 5, 8, 25, 28, 36, 39 |
| Clause 326.- 1, 15, 25, 28, 36 | Clause 614.- 1, 15, 25, 28, 36, 39 |
| Clause 327.- 1, 3, 15, 25, 28, 36 | Clause 615.- 1, 3, 15, 25, 28, 36, 39 |
| Clause 328.- 1, 5, 15, 25, 28, 36 | Clause 616.- 1, 5, 15, 25, 28, 36, 39 |
| Clause 329.- 1, 8, 15, 25, 28, 36 | Clause 617.- 1, 8, 15, 25, 28, 36, 39 |
| Clause 330.- 1, 3, 8, 15, 25, 28, 36 | Clause 618.- 1, 3, 8, 15, 25, 28, 36, 39 |
| Clause 331.- 1, 5, 8, 15, 25, 28, 36 | Clause 619.- 1, 5, 8, 15, 25, 28, 36, 39 |
| Clause 332.- 1, 21, 25, 28, 36 | Clause 620.- 1, 21, 25, 28, 36, 39 |
| Clause 333.- 1, 3, 21, 25, 28, 36 | Clause 621.- 1, 3, 21, 25, 28, 36, 39 |
| Clause 334.- 1, 5, 21, 25, 28, 36 | Clause 622.- 1, 5, 21, 25, 28, 36, 39 |
| Clause 335.- 1, 8, 21, 25, 28, 36 | Clause 623.- 1, 8, 21, 25, 28, 36, 39 |
| Clause 336.- 1, 3, 8, 21, 25, 28, 36 | Clause 624.- 1, 3, 8, 21, 25, 28, 36, 39 |
| Clause 337.- 1, 5, 8, 21, 25, 28, 36 | Clause 625.- 1, 5, 8, 21, 25, 28, 36, 39 |
| Clause 338.- 1, 15, 21, 25, 28, 36 | Clause 626.- 1, 15, 21, 25, 28, 36, 39 |
| Clause 339.- 1, 3, 15, 21, 25, 28, 36 | Clause 627.- 1, 3, 15, 21, 25, 28, 36, 39 |
| Clause 340.- 1, 5, 15, 21, 25, 28, 36 | Clause 628.- 1, 5, 15, 21, 25, 28, 36, 39 |
| Clause 341.- 1, 8, 15, 21, 25, 28, 36 | Clause 629.- 1, 8, 15, 21, 25, 28, 36, 39 |
| Clause 342.- 1, 3, 8, 15, 21, 25, 28, 36 | Clause 630.- 1, 3, 8, 15, 21, 25, 28, 36, 39 |
| Clause 343.- 1, 5, 8, 15, 21, 25, 28, 36 | Clause 631.- 1, 5, 8, 15, 21, 25, 28, 36, 39 |
| Clause 344.- 1, 39 | |

Clause 632.- The pharmaceutical composition according to any one of the preceding clauses from 57 to 631 obtained by process of clause 40, preferably obtained by the process of clause 47.

All percentages, parts and ratios herein disclosed are by weight unless specifically noted otherwise. As used herein, the term "about" refers preferably to a range that is +-10 %, preferably +-5 %, or more preferably the value with which the term is associated.

### EXAMPLES

### Example 1. Preparation of dexketoprofen trometamol.

A solution of (+)-(S)-2-(3-benzoylphenyl)propionic acid (5.0 g, 19.7 mmol) in ethanol (15 ml) is added with a solution of tromethamine (2.4 g, 19.7 mmol) in water (8 ml). The mixture is stirred at room temperature for 1/2 hour, then evaporated to dryness, to give a semi-solid residue, which is redissolved in ethanol and then evaporated to dryness, to obtain a loose solid which is crystallized from ethanol-ethyl acetate, affording 6.8 g (92%) of the title compound as a white crystalline solid, with melting point 104.8-105.1 °C.
[alfa]_{D20} = -5.2° (c=1.47, methanol).
IR (KBr): 3060, 1650, 1570, 1400, 1360, 1290, 1020, 720, 650 cm-1.
N.M.R. 1 H (300 MHz, CD3OD) 6 ppm: 1.45 (d, 3H); 3.64 (s, 6H); 3.66 (q,1IH); 7.41-7.80 (m, 9H).
Elemental analysis for C20H25NO6:
Calculated: C, 63.99%; H, 6.71%; N, 3.73%.
Found : C, 63.60% ; H, 6.40%; N, 3.73%

### Example 2. Preparation of 3 batches of dexketoprofen trometamol oral solution 25mg/10ml

| **Ingredient** | **mg/vial 10 ml** Laboratory Batch | **g/batch 150 l** Batch n° 12052(*) |
|---|---|---|
| Dexketoprofen trometamol | 36.9 | 553.50 |
| Polyethylene glycol 400 (Polyglycol 400) | 750.0 | 11250.00 |
| Methyl paraben | 20.0 | 304.20 |
| Neohesperidin Dihydrochalcone | 1.5 | 22.50 |
| Ammonium Glycyrrhizate | 2.0 | 30.00 |
| Saccharose | 2000.0 | 30000.00 |
| Sodium Saccharin | 2.0 | 30.00 |
| Sodium dihydrogen phosphate dihydrate | 45.6 | 684.00 |
| di-Sodium Hydrogen Phosphate anh. | 10.4 | 156.00 |
| Povidone (Plasdone K90) | 500.0 | 7500.00 |
| Lemon Natural Flavour | 20.0 | 300.0 |
| Purified water | q.s | q.s |

| | | |
|---|---|---|
| (*) Same quantities were used for Pilot Batch n° 12053 | | |

Manufacturing Process (batch size 15,000 units for Pilot Batches n° 12052 and n° 12053):
1. Polyethylene glycol 400, methyl paraben, neohesperidin dihydrochalcone and ammonium glycyrrhizate and water were mixed at 50 °C of temperature and the solution was stirred for approximately 15 min, to prepare a first mixture;
2. The first mixture was diluted with 105.5 l of water at a temperature between 17°C and 24°C.
3. The temperature of the diluted mixture prepared in step (2) was 24 °C;
4. Dexketoprofen trometamol, saccharose, sodium saccharin, sodium dihydrogen phosphate dihydrate, di-sodium hydrogen phosphate anhydrous, povidone and lemon natural flavour were added to the mixture prepared in step (3);
5. The solution was stirred until a filling solution was obtained;
6. The measure of the solution's pH was 6.14;
7. The filling solution was packaged at a temperature of 24 °C.

The process for the laboratory batch (batch size 10 units) was analogous to the one described above.

### Example 3. Determination of viscosity of Dexketoprofen oral solution 25 mg/10 ml.

Viscosity is a measure of a fluid's resistance to flow. The principle of the method is to measure the force acting on a rotor (torque) when it rotates at a constant angular velocity (rotational speed) in a liquid. Rotating viscometers are used for measuring the viscosity of Newtonian (shear-independent viscosity) or non-Newtonian liquids (shear dependent viscosity or apparent viscosity).

The viscosity is measured by the method described in European Pharmacopoeia 7.0^{th} edition 2.2.10. (Viscosity - Rotating viscometer method).

Apparatus: Viscometer Brookfield mod. LVDVE with spindle (ref. 61)

### Viscosity results:

- Pilot batch (n° 12052). Viscosity = 179.7 cP (temp. 21.3 °C). 20 rpm. 59.8% effort.
- Pilot batch (n° 12053). Viscosity = 126 cP (temp. 23.0 °C). 20 rpm. 42.1% effort.

### Example 4. Stability Studies for Pilot Batch n° 12052 - Impurity profile

| **Batch N° 12052** | **t=0** | **25 °C / 60% RH- 3 month** | **30 °C / 65% RH - 3 month** | **40 °C / 75% RH- 3 month** |
|---|---|---|---|---|
| **Appearance** | transparent solution | transparent solution | transparent solution | transparent solution |
| **pH** | 6.15 | 6.14 | 6.13 | 6.11 |
| **Methyl paraben content** (%) | 100.6 | 100.6 | 101.4 | 100.5 |
| **Assay (%)** | 99.4 | 98.9 | 98.6 | 99.1 |
| **RELATED SUBSTANCES** Unknown individual impurities (%) | 0.10 | 0.33 | 0.26 | 0.47 |
| **Total impurities** (%) | 0.10 | 0.81 | 0.97 | 1.30 |

## Claims

1. A pharmaceutical composition in the form of stick-pack comprising dexketoprofen trometamol, wherein the pharmaceutical composition comprises at least the following two portions:
i) a filling solution, wherein the filling solution comprises dexketoprofen trometamol and at least a pharmaceutically acceptable excipient or excipients, wherein the dexketoprofen trometamol is dissolved in the filling solution and the pH of the filling solution is lower than 9;
ii) a stick package, wherein the stick package comprises at least a sealed laminated foil of aluminium of less than 20.0 microns thick.
wherein the pharmaceutical composition is manufactured by the process comprising at least the following steps:
a) having a first mixture of at least a pharmaceutically acceptable excipient or excipients, and water at a temperature higher than 35 °C, preferably between 36 and 75 °C, more preferably between 43 and 60 °C, wherein the pharmaceutically acceptable excipient or excipients being preferably selected from viscosity agents, preservatives, sweetness enhancers and mixtures thereof;
b) optionally, diluting the first mixture with water;
c) preferably keeping or, if necessary, cooling the temperature of the first mixture in step (a) or of the diluted mixture in step (b) below 45 °C, more preferably below 35 °C,
d) adding dexketoprofen trometamol, or a mixture of dexketoprofen trometamol and a further pharmaceutically acceptable excipient or excipients, to the mixture of step (a), (b) or (c);
e) stirring the mixture obtained in the preceding step;
f) optionally, measuring the pH of the filling solution, and adjusting to a pH below 9 if required;
g) packaging the filling solution in a stick package, wherein the stick package comprises at least a sealed laminated foil of aluminium of less than 20.0 microns thick;
optionally, at least 15,000 units of pharmaceutical composition are manufactured.

2. A pharmaceutical composition in the form of stick-pack comprising dexketoprofen trometamol, wherein the pharmaceutical composition comprises at least the following two portions:
i) a filling solution, wherein the filling solution comprises dexketoprofen trometamol and at least a pharmaceutically acceptable excipient or excipients, wherein the dexketoprofen trometamol is dissolved in the filling solution and the pH of the filling solution is lower than 9;
ii) a stick package, wherein the stick package comprises at least a sealed laminated foil of aluminium of less than 20.0 microns thick.

3. The pharmaceutical composition according to the preceding claim, wherein the sealed laminated foil of aluminium of the stick package is from 5 to 14.0 microns thick, preferably from 7 to 12.0 microns thick, more preferably less than 11.0 microns thick, even more preferably less than 10.0 microns thick, and even yet more preferably is about 9 microns thick; or wherein the pH of the filling solution is about 3 to 8, preferably is about 4.0 to 7.5, and more preferably is about 5.5 to 6.5.

4. The pharmaceutical composition according to any one of the preceding claims, wherein the stick package: comprises at least an internal layer and preferably the internal layer is of polyethylene; comprises at least an external layer and preferably the external layer is of polyethylene; comprises at least an external layer and at least an internal layer and preferably the stick package comprises at least a polyethylene external layer and at least a polyethylene internal layer; comprises at least an outer layer and preferably the outer layer is printed paper or polyethylene terephthalate; or consists essentially of printed paper foil/polyethylene foil/aluminium foil/polyethylene foil or polyethylene terephthalate foil/polyethylene foil/aluminium foil/polyethylene foil.

5. The pharmaceutical composition according to any one of the preceding claims, wherein the concentration of dexketoprofen trometamol is about 0.5 to 10 mg/ml, preferably is about 1 to 5 mg/ml, and more preferably is about 2.5 mg/ml.

6. The pharmaceutical composition according to any one of the preceding claims, comprising at least a viscosity agent, at least a preservative, at least a sweetness enhancer and, if required at least a pH modifier agent, preferably the viscosity agent is selected from povidone, polyethylene glycol, and mixtures thereof.

7. The pharmaceutical composition according to the preceding claim, wherein: the total amount of amount of viscosity agent or viscosity agents, preferably povidone, polyethylene glycol and mixtures thereof, is below 60% w/w, preferably from 15 to 45% w/w in respect of the total amount of the pharmaceutical composition; wherein the pharmaceutical composition comprises povidone, the amount of povidone is below 40% w/w, preferably from 12 to 25% w/w in respect of the total amount of the pharmaceutical composition; or wherein the pharmaceutical composition comprises polyethylene glycol, the amount of polyethylene glycol is below 40% w/w, preferably from 15 to 30% w/w in respect of the total amount of the pharmaceutical composition.

8. The pharmaceutical composition according to any one of the two preceding claims, wherein:_the preservative is selected from salicylic acid, benzoic acid, methyl paraben, ethyl paraben, cresol, cetrimide, citric acid, EDTA and mixtures thereof, preferably the preservative agent is methyl paraben; or wherein the amount of preservative agent, preferably methyl paraben, is_below 4% w/w, preferably ranges from 2 to 0.2% w/w, more preferably from 1 to 0.4% w/w in respect of the total amount of the pharmaceutical composition.

9. The pharmaceutical composition according to any one of the three preceding claims, wherein: the sweetness enhancer is neohesperidin dihydrochalcone, sodium saccharin, ammonium glycyrrhizate or mixtures thereof; or wherein the total amount of sweetness enhancer or sweetness enhancers, preferably neohesperidin dihydrochalcone, sodium saccharin, ammonium glycyrrhizate and mixtures thereof, is below 2% w/w, preferably below 1% w/w, more preferably below 0.3% w/w in respect of the total amount of the pharmaceutical composition.

10. The pharmaceutical composition according to any one of the four preceding claims, wherein: the pH modifier agent is sodium phosphate, preferably the pH modifier agent is di-sodium hydrogen phosphate, monosodium dihydrogen phosphate or mixtures thereof; or the total amount of pH modifier agent or pH modifier agents, preferably di-sodium hydrogen phosphate, monosodium dihydrogen phosphate and mixtures thereof, is below 8% w/w, preferably ranges from 0.1 to 5% w/w, more preferably from 1 to 3% w/w in respect of the total amount of the pharmaceutical composition.

11. A process for the manufacture of an pharmaceutical composition according to any one of the preceding claims, wherein the process comprises at least the following steps:
a) having a first mixture of at least a pharmaceutically acceptable excipient or excipients, and water at a temperature higher than 35 °C, preferably between 36 and 75 °C, more preferably between 43 and 60 °C, wherein the pharmaceutically acceptable excipient or excipients being preferably selected from viscosity agents, preservatives, sweetness enhancers and mixtures thereof;
b) optionally, diluting the first mixture with water;
c) preferably keeping or, if necessary, cooling the temperature of the first mixture in step (a) or of the diluted mixture in step (b) below 45 °C, more preferably below 35 °C,
d) adding dexketoprofen trometamol, or a mixture of dexketoprofen trometamol and a further pharmaceutically acceptable excipient or excipients, to the mixture of step (a), (b) or (c);
e) stirring the mixture obtained in the preceding step;
f) optionally, measuring the pH of the filling solution, and adjusting to a pH below 9 if required;
g) packaging the filling solution in a stick package, wherein the stick package comprises at least a sealed laminated foil of aluminium of less than 20.0 microns thick;
optionally, at least 15,000 units of pharmaceutical composition are manufactured.

12. The process for the manufacture of a pharmaceutical composition according to the preceding process claim, wherein the process comprises at least the following steps:
a) having a first mixture of polyethylene glycol 400, methyl paraben, neohesperidin dihydrochalcone and ammonium glycyrrhizate, and water at a temperature higher than 35 °C, preferably between 36 and 70 °C;
b) optionally diluting the first mixture with water;
c) preferably keeping or, if necessary, cooling the temperature of the first mixture prepared in step (a) or of the diluted first mixture prepared in step (b) below 45 °C;
d) adding dexketoprofen trometamol, saccharose, sodium saccharin, sodium dihydrogen phosphate dihydrate, di-sodium hydrogen phosphate anydrous, povidone and lemon natural flavour to the mixture of step (a), (b) or (c);
e) stirring the mixture obtained in the preceding step;
f) optionally, measuring the pH of the solution, and adjusting to a pH between 5.7 to 6.5 if required;
g) packaging the filling solution in a stick package, wherein the stick package comprises at least a sealed laminated foil of aluminium of less than 20.0 microns thick.

13. A filling solution comprising dexketoprofen trometamol, wherein the dexketoprofen trometamol is dissolved, the pH of the filling solution is lower than 9, and wherein the filling solution is obtained by the process as defined in the steps of any of the preceding process claims, preferably the filling solution is packaged in a stick package as defined in the above portion (ii) of the preceding product claims.

14. A pharmaceutical batch of at least 15,000 units of the pharmaceutical composition according to any one of the preceding product claims.

15. A cardboard box with patient information leaflet in pack size of at least 4 stick-pack as defined in any one of the preceding product claims.
